Europäisches Patentamt

European Patent Office

Office européen des brevets

(19)

(11) **EP 0 659 081 B1**

(12) **EUROPÄISCHE PATENTSCHRIFT**

(45) Veröffentlichungstag und Bekanntmachung des Hinweises auf die Patenterteilung:
**11.03.1998 Patentblatt 1998/11**

(21) Anmeldenummer: 93919070.8

(22) Anmeldetag: **16.08.1993**

(51) Int. Cl.$^6$: **A61K 31/565**, A61K 9/70

(86) Internationale Anmeldenummer:
**PCT/EP93/02182**

(87) Internationale Veröffentlichungsnummer:
**WO 94/06436** (31.03.1994 Gazette 1994/08)

(54) **DEXPANTHENOL-HALTIGES PFLASTER ZUR TRANSDERMALEN APPLIKATION VON STEROIDHORMONEN**

DEXPANTHENOL-CONTAINING PLASTER FOR THE TRANSDERMAL APPLICATION OF STEROID HORMONES

EMPLATRE CONTENANT DU DEXPANTHENOL POUR APPLICATION TRANSCUTANEE D'HORMONES STEROIDES

(84) Benannte Vertragsstaaten:
**AT BE CH DE DK ES FR GB GR IE IT LI LU NL PT SE**

(30) Priorität: **12.09.1992 DE 4230588**

(43) Veröffentlichungstag der Anmeldung:
**28.06.1995 Patentblatt 1995/26**

(73) Patentinhaber:
**LTS LOHMANN Therapie-Systeme GmbH**
**56567 Neuwied (DE)**

(72) Erfinder: **ROREGER, Michael**
**D-56566 Neuwied (DE)**

(74) Vertreter:
**Flaccus, Rolf-Dieter, Dr.**
**Patentanwalt**
**Sperlingsweg 32**
**50389 Wesseling (DE)**

(56) Entgegenhaltungen:
**EP-A- 0 279 977          EP-A- 0 380 989**
**WO-A-86/01402**

**Beschreibung**

Die Erfindung betrifft ein selbstklebendes Pflaster zur transdermalen Applikation systemisch wirksamer Steroidhormone, mit einer wirkstoffundurchlässigen Rückschicht, einer wirkstoffhaltigen Haftklebeschicht und einer diese vor der Applikation auf der Haut abdeckenden ablösbaren Schutzschicht.

Wirkstoffpflaster als therapeutische Systeme (TTS) sind auf die Haut aufzubringende selbstklebende Vorrichtungen mit definierter Applikationsfläche, die einen oder mehrere darin enthaltene Arzneistoffe nach Zeit und Menge kontrolliert an den menschlichen oder tierischen Körper abgeben.

Voraussetzung für die transdermale Wirkstoffaufnahme ist allerdings, daß der Wirkstoff in der galenischen Zubereitung in einer Form bereitgestellt wird, die seine Aufnahme in die Haut ermöglicht, daß heißt, der Wirkstoff muß in der Zubereitung zumindest teilweise gelöst sein. Von den Steroidhormonen und insbesondere von dem aus dieser Gruppe für die transdermale Verabreichung am gründlichsten untersuchten Wirkstoff Estradiol ist bekannt, daß sie nur eine sehr geringe Löslichkeit in den Formulierungen aufweisen, die üblicherweise für transdermale Wirkstoffabgabe geeignet sind und verwendet werden. Bei diesen Formulierungen, bei denen als Basispolymere z.B. Polyacrylate oder Polyisobutylene eingesetzt werden, tritt in der Regel während der Lagerung der Pflaster eine Rekristallisation des Wirkstoffs auf, die das Freisetzungs- und damit das Resorptionsverhalten verändert.

Es besteht grundsätzlich die Möglichkeit, durch den Einsatz von Lösungsvermittlern die Löslichkeit des Wirkstoffs während der Lagerung zu erhalten und die Rekristallisation zu verhindern.

Bei den im Markt eingeführten Estradiol-TTS mit dem Handelsnamen "Estraderm" werden Estradiol und Ethanol als Lösemittel für Estradiol in einer Pflasterformulierung verabreicht, bei der die Wirkstofflösung in einem Beutel enthalten ist (EP 0 285 563). Nachteilig bei diesem Pflaster sind zum einen der komplizierte Aufbau und die aufwendige Herstellung, zum anderen die Tatsache, daß insbesondere bei mehrtägiger Applikation Hautreizungen auftreten können, die auf den Lösungsvermittler Ethanol zurückzuführen sind.

Bei herkömmlich aufgebauten transdermalen therapeutischen Systemen mit z.B. einschichtigen, selbstklebenden Wirkstoffmatrices führt der Einsatz von Lösungsvermittlern in der mindestens benötigten Konzentration zu Kohäsionsverlusten, das heißt zum "Matschigwerden" der Matrices. Das gilt insbesondere für Dexpanthenol, eine bei Raumtemperatur hochviskose Flüssigkeit, die in üblichen Formulierungen bereits in recht niedrigen Konzentrationen einen stark weichmachenden Einfluß ausübt. In der EP 0 380 989 ist Dexpanthenol als Penetrationshilfsmittel bei der transdermalen Verabreichung von systemisch wirksamen pharmazeutischen Wirkstoffen beschrieben. Dabei umfaßt die Matrixmasse insbesondere Polymere auf Acrylatbasis, die allerdings auch andere Comonomere einpolymerisiert enthalten können, unter denen auch Vinylacetat genannt wird. Die Verarbeitung erfolgt vornehmlich unter Bildung einer wäßrigen Latexdispersion meist mit Zusatz erheblicher Ethanolmengen. Als besonderer Vorteil der Verwendung von Dexpanthenol wird angegeben, daß Dexpanthenol nicht nur penetrationsfördernd wirkt, sondern auch hautirritierende Einflüsse von Wirk- und Hilfsstoffen unterdrückt oder vermindert.

Durch eigene Untersuchungen wurde nun ermittelt, daß Dexpanthenol ein gutes Lösemittel bzw. ein guter Lösungsvermittler für Steroidhormone ist und insbesondere in Haftschmelzklebermassen zu günstigen Pflastereigenschaften beiträgt.

Aufgabe der vorliegenden Erfindung war es daher, eine Applikation von Steroidhormonen für ein Pflaster zur transdermalen Formulierung anzugeben, die es erlaubt, Dexpanthenol in den für die Lösungsvermittlung mindestens erforderlichen Konzentration in die Formuliervg einzuarbeiten, ohne daß es dadurch zu Kohäsionsverlusten und zu einem Zerfließen der wirkstoffhaltigen Pflasterkomponente bei der Applikation kommt.

Das erfindungsgemäße selbstklebende Pflaster der eingangs genannten Art ist demgemäß dadurch gekennzeichnet, daß die wirkstoffhaltige Haftklebeschicht einen Ethylen-Vinylacetat-Copolymer mit einem Vinylacetatgehalt von 20 bis 35 % als Polymer enthaltenden Haftschmelzkeber mit einer Verarbeitungstemperatur zwischen 60 und 100 °C und Dexpanthenol in einer Konzentration von 15 bis 25 Gew.-% enthält.

Da üblicherweise Formulierungen zur transdermalen Applikation systemisch wirksamer Steroidhormone das betreffende Steroidhormon, z.B. Estradiol, in einer Konzentration von 1 bis 2 % enthalten, ist unter Berücksichtigung der jeweiligen Sättigungslöslichkeit des Steroidhormons in Dexpanthenol ein Anteil von mindestens 15 Gew.-% Dexpanthenol erforderlich, um ein Auskristallisieren des Wirkstoffes in der Formulierung sicher zu verhindern. Trotz gegenteiliger Erwartungen ist es möglich, diese Konzentration an Dexpanthenol in einer wirkstoffhaltigen Formulierung für die transdermale Applikation zu erreichen, ohne daß es zu einem Verlust des inneren Zusammenhaltes der haftklebenden Formulierung kommt, wenn ein Haftschmelzkleber mit einer Verarbeitungstemperatur von 60 bis 100 °C gewählt wird.

Haftschmelzkleber sind feste, thermoplastische Formulierungen, die im wesentlichen Rohstoffe aus der Gruppe der Polymeren, der Harze, der Füllstoffe und der Alterungsschutzmittel enthalten.

Die erfindungsgemäßen Haftschmelzkleber der dexpanthenolhaltigen Haftkleberschicht enthalten als Polymere Ethylen-Vinylacetat-Copolymere mit 20 bis 35 % Vinylacetat und werden bei Temperaturen zwischen 60 und 100 °C in geschmolzenem Zustand verarbeitet und erstarren unter Aufbau adhäsiver und kohäsiver Kräfte, wobei letztere in der Regel umso geringer sind, je niedriger die Erweichungstemperatur des Haftschmelzklebers ist.

2

EP 0 659 081 B1

Dabei können -soweit die vorstehende Forderung erfüllt ist - als Polymere gegebenenfalls auch Homo-, Co- oder Block- polymere wie z.B. Polyamide, Polyester, Polycaprolactame, Polycaprolacton, Ethylen- Ethylacrylat-Copolymere (EEA), Polyvinylether, Poly(meth)acrylate, Polyvinylacetale, Polyvinylacetate, Styrol-Butadien-Blockpolymere, Isopren-Blockpolymere, Polyurethane, Ethylcellulose, Celluloseacetat-Butyrat, Synthesekautschuke (z.B. Neopren-Kautschuk), Polyisobutylen, Butylgummi, Acrylnitril-Butadien-Mischpolymerisate, Epoxidharze, Melaminharze, Phenol-Formaldehyd-Harze und Resorcin-Formaldehyd-Harze verwendet werden, wobei auch unter anderem die nachfolgend genannten modifizierten Harze eingesetzt werden können:

Hydriertes Kolophonium, polymerisiertes Kolophonium, dimerisierte Harzsäuren, disproportioniertes Kolophonium, Methylester von Kolophonium, Glycerinester von hydriertem Kolophonium, Methylester von hydriertem Kolophonium, Pentalester, Triethylenglykolester von hydriertem Kolophonium, Hydroabietylalkohol und dessen Derivate, Glycerinester, Di-Triolester und Pentalester von Harzsäuren, Pentalester von polymerisiertem Kolophonium, Pentalester von dimerisiertem Kolophonium, Glycerinester von dimerisiertem Kolophonium, Ester von Maleinsäure- oder Phenolmodifiziertem Kolophonium, aromatische und aliphatische Kohlenwasserstoffharze, hydrierte Harze, Polyterpenharze, modifizierte Terpenharze, Wachse, niedermolekulares Polyethylen und Polypropylen, Alkyl-Styrol-Copolymere. Außerdem können Füllstoffe, wie z.B. Titandioxid, Magnesiumoxid, Zinkoxid und Silciumdioxid sowie Alterungsschutzmittel wie z.B. Tocopherol, substituierte Phenole, Hydrochinone, Brenzkatechine und aromatische Amine zugemischt werden.

Die Erweichungstemperatur wird insbesondere durch sogenannte weichmachende Substanzen, zu denen auch Dexpanthenol zählt, erniedrigt, wobei in der Regel bereits geringe Konzentrationen an Weichmachern zu deutlichen Köhasionverlusten führen. Es wurde bereits erwähnt, daß der Weichmacher Dexpanthenol überraschenderweise in ungewöhnlich hohen Konzentrationen in Haftschmelzkleberformulierungen eingearbeitet werden kann, ohne daß die erwähnten Kohäsionsverluste beobachtet werden. Allerdings sind auch der Weichmacheraufnahmekapazität einer erfindungsgemäßen Haftschmelzkleberformulierung mit einer Verarbeitungstemperatur von 60 bis 100 °C Grenzen gesetzt.

Oberhalb eines Dexpanthenol-Anteils von 25 Gew.-% kommt es auch bei den vorgenannten Haftschmelzkleberformulierungen zu einer Erniedrigung der Erweichungstemperatur in einem Bereich nahe der Körpertemperatur, so daß es bei Applikation derartiger Formulierungen zu einem unerwünschten Erweichen des Haftschmelzklebers auf der Haut kommt.

Bei der Formulierung wirkstoffhaltiger Haftschmelzkleber zur transdermalen Applikation systemisch wirksamer Steroidhormone ist zu beachten, daß die zur Herstellung und Verarbeitung des Haftschmelzklebers erforderlichen Temperaturen in einem Rahmen gehalten werden, in dem es nicht zu unerwünschten Veränderungen des Steroidhormons oder anderer Formulierungsbestandteile kommt. Die Temperaturstabilität der bekannten Steroidhormone ist bei Temperaturen kleiner 100 °C recht gut, so daß eine Haftschmelzkleberformulierung für Steroidhormone idealerweise eine Verarbeitungstemperatur von kleiner 100 °C aufweist. Andererseits sollte die Verarbeitungstemperatur größer 60 °C sein, damit eine ausreichende Kohäsion des Haftschmelzklebers nach dem Erhalten erzielt wird.

Besonders geeignet zur Erfüllung der Anforderungen bezüglich Verarbeitungstemperatur (60 bis 100 °C), Kohäsion der Formulierung (max. 25 Gew.-% Dexpanthenol) und Löslichkeit des Steroidhormons (mind. 15% Dexpanthenol) ist ein dexpanthenolhaltiger Haftschmelzkleber der Zusammensetzung:

10 bis 30 Gew.-% Ethylen-Vinylacetat-Copolymer mit einem Vinylacetatgehalt von 28%

15 bis 45 Gew.-% Weichharz mit einer Schmelzviskosität kleiner 10 Pa • s bei 60°C

10 bis 30 Gew.-% aliphatisches Kohlenwasserstoffharz mit einer Schmelzviskosität größer 10 Pa • s bei 100 °C

15 bis 25 Gew.% Dexpanthenol

0,1 bis 10 Gew.-% Steroidhormon

0 bis 5 Gew.-% Füllstoff

0 bis 5 Gew.-% Alterungsschutzmittel

Eine Haftschmelzkleberformulierung der genannten Zusammensetzung kann z.B. enthalten

16,7 Gew.-% Ethylen-Vinylacetat-Copolymer mit einem Vinylacetatgehalt von 28%

35,8 Gew.-% Hydroabietylalkohol

23,2 Gew.-% Polyalkadien (Monomerkettenlänge C4 bis C5)

21,6 Gew.-% Dexpanthenol

0,7 Gew.-% Siliciumdioxid

2,0 Gew.-% Estradiol

Wird diese Formulierung nach dem Aufschmelzen und Homogenisieren bei einer Temperatur von beispielsweise 90 °C auf ein Trägermaterial ausgestrichen, wird ein homogener, transparenter Film erhalten, in dem auch 12 Monate nach Herstellung bei mikroskopischen Untersuchungen keine Estradiolkristalle aufgefunden werden.

Wird eine Haftschmelzkleberformulierung gleicher Zusammensetzung, jedoch ohne Zusatz von Dexpanthenol, hergestellt, so kann der einzuarbeitende Wirkstoff Estradiol nicht vollständig gelöst werden. Nach dem Ausstreichen und Erkalten des Haftschmelzklebers kommt es schnell zur Auskristallisation des Wirkstoffs aus der übersättigten Lösung und zu fortschreitendem Kristallwachstum insbesondere dort, wo sich bereits Wirkstoffkristalle befinden, die sich in der Schmelze nicht gelöst haben.

Das Beispiel macht zum einen die besondere Eignung von Dexpanthenol für die Lösungsvermittlung deutlich, wenn Steroidhormone in Haftschmelzklebern mit einer Verarbeitungstemperatur von 60 bis 100 °C gelöst werden sollen; zum anderen zeigt das Beispiel die Fähigkeit von Dexpanthenol, den Zustand der übersättigten Lösung von Steroidhormonen in der Haftschmelzkleberformulierung zu stabilisieren und das Rekristallisieren des Steroidhormons und damit die Destabilisierung des Systems zu verhindern.

Systemisch wirksame Steroidhormone gemäß der Erfindung können z.B. Östrogene sein. Dazu zählen das wirksamste natürlich vorkommende Östrogen, daß 17 β-Estradiol, sowie Ester, Ether oder Ethinylverbindungen des Estradiols, wie z.B.

- Estradiol (17β)-17-Butyrylacetat,

- Estradiol 17 β-cipionat,

- Estradiol 3,17 β-dienantat,

- Estradiol 3,17 β-dipropionat,

- Estradiolenantat,

- Estradiol 3-hydrogensulfat (Natriumsalz),

- Estradiol 17 β-(3-phenylpropionat),

- Estradiolundecylat

- Estradiolvalerat,

- Estradiol 17 -(oxohexonat),

- Epimestrol,

- Quinestrol,

- Quinestradol,

- Ethinylestradiol,

- Fosferol, sowie

- Estratriol.

Desweiteren können systemisch wirksame Steroidhormone gemäß der Erfindung Gestagene sein, wie z.B.:

- Lynestrenol,

- Norethisteron,

- Hydroxyprogesteron,

- Progesteron,

- Medroxyprogesteron,

- Gestonoron,

- Dydrogesteron,

- Chlormadinon,

- Allylestrenol,

- Megestrol,

- Medrogeston

Dexpanthenolhaltige Pflaster zur transdermalen Applikation von systemisch wirksamen Steroidhormonen, bei denen der wirkstoffabgebende Teil der Pflaster Haftschmelzkleber enthält, können nach allen dem Fachmann bekannten Pflasterkonstruktionen aufgebaut sein, so z.B. nach dem Matrixsystem oder dem Membransystem.

Ein Membransystem enthält mindestens 5 Teile: eine flexible Rückschicht, einen wirkstoffhaltigen Haftschmelzkleber, eine Membran zur Steuerung der Wirkstofffreisetzung, eine auf die Membran laminierte Klebschicht zur Befestigung des Systems auf der Haut sowie einer ablösbaren Schutzschicht, die die zur Haut gewandte, klebende Fläche des Pflasters abdeckt. Ein Matrixsystem enthält in einfachster Weise drei Teile: eine flexible Rückschicht, einen wirkstoffhaltigen Haftschmelzkleber und eine ablösbare Schutzschicht, die die zur Haut gewandte, klebende Fläche des Pflasters abdeckt.

Geeignete Materialien für die Rückschicht sind beispielsweise Polyester, Polyamid, Polyethylen, Polypropylen, Polyurethan, Polyvinylchlorid, und zwar sowohl als sogenannte Solofolien als auch als Sandwichfolien in Kombination von Folien aus verschiedenen dieser Kunststoffe. Diese Folien können außerdem mit Aluminium bedampft bzw. mit einer Aluminiumfolie kaschiert sein.

Geeignete Materialien für die ablösbare Schutzschicht sind beispielsweise Polyester, Polyethylen und Polypropylen sowie Papiere, die mit diesen Materialien beschichtet und ggf. mit Aluminium bedampft bzw. mit einer Aluminiumfolie kaschiert wurden. Außerdem sind die Folien bzw. Papiere mit Silikon beschichtet, um ihnen die wiederablösbaren Eigenschaften zu verleihen.

Der wirkstoffabgebende Haftschmelzkleber kann in einer oder mehreren Schichten vorliegen, Im letzteren Fall können die Schichten unterschiedlich zusammengesetzt sein. Dabei soll durch Verwendung unterschiedlicher Hilfsstoffe oder unterschiedlicher Wirkstoffkonzentrationen in den einzelnen Schichten ein Freisetzungsverhalten des Wirkstoffes erreicht werden, das für die Anwendung notwendig und durch einschichtigen Aufbau z.B. nicht zu erzielen ist.

Ein Verfahren zur Herstellung eines erfindungsgemäßen Pflasters ist entsprechend den folgenden Merkmalen von Anspruch 9 vorgesehen:

a) Harzanteile der Formulierung werden bei ca. 60 bis 200 °C geschmolzen,

b) in die Schmelze werden Polymere bis zur klaren Schmelze langsam eingerührt,

c) nach oder zugleich werden Hilfs- und/oder Füllstoffe langsam eingerührt, bis eine gleichmäßige Verteilung erreicht ist (Schmelze A),

d) in einem separaten Rührgefäß wird Dexpanthenol bei 50 °C geschmolzen, in die Schmelze wird Estradiol portionsweise langsam eingerührt und zwar, bis zur klaren Lösung (Schmelze B)

e) Schmelze B wird langsam in Schmelze A eingerührt bis eine gleichmäßige Durchmischung erreicht ist,

5

f) der Haftschmelzkleber wird dann bei 60 bis 100 °C mittels Rakelauftrag mit einem Flächengewicht von vorzugsweise 80 bis 500 g/m$^2$, vorzugsweise 150 bis 280 g/m$^2$, auf eine silikonisierte Polyester-Schutzschicht (Stärke 30 bis 250 μm) gestrichen, nach dem Erkalten wird eine Polyester-Rückschicht (Stärke 6 bis 100 μm) auf den Haftschmelzkleber laminiert,

g) anschließend werden Pflaster mit einer Fläche von 10 cm$^2$ ausgestanzt.

Die Verarbeitung der wirkstoffabgebenden Haftkleberschicht des Pflasters, welche Haftschmelzkleber mit einer Verarbeitungstemperatur zwischen 60 und 100 °C aufweist, kann durch bekannte Verfahren wie z.B. Extrusion, Gießen, Walzenauftrag, Rakelauftrag, Aufsprühen oder ein Druckverfahren erfolgen.

Beispiele:

| | 1 | 2 |
|---|---|---|
| Ethylen-Vinylacetat-Copolymer mit einem Vinylacetatgehalt von 28% | 17,7 g | 16,7 g |
| Dexpanthenol | 15,9 g | 21,6 g |
| Hydroabietylalkohol | 38,9 g | 35,8 g |
| Polyalkadien (Monomerkettenlänge C4 bis C5) | 24,7 g | 23,2 g |
| Siliciumdioxid | 0,7 g | 0,7 g |
| Ethylcellulose | 0,7 g | |
| Estradiol | 1,4 g | 2,0 g |
| | 100,0 g | 100,0 g |

| | 1 | 2 |
|---|---|---|
| Flächengewicht der wirkstofffreien Haftklebeschicht (g/m$^2$) | 230 | 231 |
| Wirkstoffgehalt des Pflasters (mg/10 cm$^2$) | 2,80 | 4,24 |
| Mäusehautpenetration (μg/10 cm$^2$) | | |
| 8 h | 33 | 38 |
| 24 h | 91 | 113 |
| 48 h | 257 | 340 |
| In vitro-Freisetzung (paddle over disc; μg/10 cm$^2$) | | |
| 4 h | 116 | 194 |
| 24 h | 430 | 752 |

Die Herstellung der Pflaster nach den Formulierungsbeispielen 1 und 2 erfolgt dergestalt, daß beispielsweise Hydroabietylalkohol bei 90 °C geschmolzen wird. In die Schmelze werden Ethylen-Vinylacetat-Copolymer und Polyalkadien bis zur klaren Schmelze langsam eingerührt. Danach werden Siliciumoxid und, in Beispiel 1, Ethylcellulose langsam eingerührt, bis eine gleichmäßige Verteilung erreicht ist (Schmelze A).

In einem separaten Rührgefäß wird Dexpanthenol bei 50 °C geschmolzen. In die Schmelze wird Estradiol portionsweise langsam eingerührt bis zur klaren Lösung (Schmelze B).

Schmelze B wird langsam in Schmelze A eingerührt, bis eine gleichmäßige Durchmischung erreicht ist.

Der Haftschmelzkleber wird dann mittels Rakelauftrag mit einem Flächengewicht von 230 g/m$^2$ auf eine silikonisierte Polyester-Schutzschicht (Stärke 100 μm) gestrichen. Nach dem Erkalten wird eine Polyester-Rückschicht (Stärke 15 μm) auf den Haftschmelzkleber laminiert.

Anschließend werden Pflaster mit einer Fläche von 10 cm$^2$ ausgestanzt.

Die Untersuchungen dieser Pflaster bezüglich der Penetration durch exzidierte Mäusehaut und der in vitro-Freiset-

EP 0 659 081 B1

zung zeigen, daß mit dem erfindungsgemäßen Pflaster optimale Penetrationswerte erzielt werden, ohne daß eine Kristallation des Wirkstoffes eintritt.

**Patentansprüche**

1. Selbstklebendes Pflaster zur transdermalen Applikation systemisch wirksamer Steroidhormone, mit einer wirkstoffundurchlässigen Rückschicht, einer wirkstoffhaltigen Haftklebeschicht und einer diese vor der Applikation auf der Haut abdeckenden ablösbaren Schutzschicht, dadurch gekennzeichnet, daß die wirkstoffhaltige Haftklebeschicht einen Ethylen-Vinylacetat-Copolymer mit einem Vinylacetatgehalt von 20 bis 35 % als Polymer enthaltenden Haftschmelzkleber mit einer Verarbeitungstemperatur zwischen 60 und 100 °C und Dexpanthenol in einer Konzentration von 15 bis 25 Gew.-% enthält.

2. Pflaster nach Anspruch 1, dadurch gekennzeichnet, daß das Steroidhormon Estradiol ist.

3. Pflaster nach Anspruch 1 oder 2, dadurch gekennzeichnet, daß der Haftschmelzkleber Weichharze, Hartharze, Füllstoffe und Alterungsschutzmittel als Hilfsstoffe enthält.

4. Pflaster nach einem oder mehreren der Ansprüche 1 bis 3, gekennzeichnet durch, eine Zusammensetzung des Haftschmelzklebers mit folgenden Bestandteilen:

    10 bis 30 Gew.-% Ethylen-Vinylacetat-Copolymer mit einem Vinylacetatgehalt von 28 %,

    15 bis 45 Gew.-% Weichharz mit einer Schmelzviskosität kleiner 10 Pa · s bei 60° C,

    10 bis 30 Gew.-% aliphatisches Kohlenwasserstoffharz mit einer Schmelzviskosität größer 10 Pa · s bei 100°C,

    15 bis 25 Gew.-% Dexpanthenol,

    0,1 bis 10 Gew.-% Steroidhormon,

    0 bis 5 Gew.-% Füllstoff,

    0 bis 5 Gew.-% Alterungsschutzmittel,

    wobei die Summe der Prozentsätze der Bestandteile 100 ist.

5. Pflaster nach einem oder mehreren der Ansprüche 1 bis 4, dadurch gekennzeichnet, daß der Haftschmelzkleber in einer oder mehreren Schichten vorliegt, wobei die einzelnen Schichten unterschiedlich zusammengesetzt sein können.

6. Pflaster nach einem oder mehreren der Ansprüche 1 bis 5, dadurch gekennzeichnet, daß es eine wirkstoffundurchlässige flexible Rückschicht, ein damit verbundenes Wirkstoffreservoir aus dem Haftschmelzkleber, bei Abwesenheit anderer Steuermechanismen eine die Abgabe des Wirkstoffs steuernde Membran, und fallweise eine mit der Membran verbundene Haftklebeeinrichtung zur Befestigung des Systems auf der Haut und eine die der Haut zugewandte, klebende Fläche des Pflasters abdeckende, vor der Applikation des Systems ablösbare Schutzschicht umfaßt.

7. Verfahren zur Herstellung des Pflasters nach den Ansprüchen 1 bis 6, gekennzeichnet durch die Arbeitsschritte:

    a) Harzanteile der Formulierung werden bei ca. 60 bis 200°C geschmolzen,

    b) in die Schmelze werden Polymere bis zur klaren Schmelze langsam eingerührt,

    c) nach oder zugleich werden Hilfs- und/oder Füllstoffe langsam eingerührt, bis eine gleichmäßige Verteilung erreicht ist (Schmelze A).

    d) in einem separaten Rührgefäß wird Dexpanthenol bei 50°C geschmolzen, in die Schmelze wird Estradiol

7

portionsweise langsam eingerührt und zwar bis zur klaren Lösung (Schmelze B)

e) Schmelze B wird langsam in Schmelze A eingerührt, bis eine gleichmäßige Durchmischung erreicht ist,

f) der Haftschmelzkleber wird dann bei 60 bis 100 °C mittels Rakelauftrag mit einem Flächengewicht von vorzugsweise 80 bis 500 g/m$^2$, vorzugsweise 150 bis 280 g/m$^2$, auf eine silikonisierte Polyester-Schutzschicht (Stärke 30 bis 250 μm) gestrichen, nach dem Erkalten wird eine Polyester-Rückschicht (Stärke 6 bis 100 μm) auf den Haftschmelzkleber laminiert.

g) anschließend werden Pflaster mit einer Fläche von 10 cm$^2$ ausgestanzt.

**Claims**

1. Self-adhesive plaster for the transdermal application of systemically active steroid hormones, comprising an active substance-impermeable backing layer, an active substance-containing pressure-sensitive adhesive layer and a removable protective layer covering said adhesive layer prior to application to the skin, characterized in that the active substance-containing pressure-sensitive adhesive layer contains a pressure-sensitive hot melt adhesive which contains as polymer an ethylene-vinylacetate copolymer having a vinyl acetate content of 20 to 35% and which has a processing temperature of between 60 and 100 °C, and dexpanthenol at a concentration of 15 to 25%-wt.

2. Plaster according to claim 1, characterized in that the steroid hormone is estradiol.

3. Plaster according to one or more of the preceding claims, characterized in that the pressure-sensitive hot melt adhesive contains as auxiliary substances flexible resins, hardened resins, fillers and ageing protecting agents.

4. Plaster according to one or more of claims 1 to 3, characterized in that the pressure-sensitive hot melt adhesive is composed of the following components:

   10 to 30%-wt. ethylene-vinylacetate copolymer having a vinyl acetate content of 28%

   15 to 45%-wt. flexible resin having a melt viscosity of below 10 Pa · s at 60 °C.

   10 to 30%-wt. aliphatic hydrocarbon resin having a melt viscosity above 10 Pa · s at 100 °C

   15 to 25%-wt. dexpanthenol

   0.1 to 10%-wt. steroid hormone

   0 to 5%-wt. filler

   0 to 5%-wt. ageing protecting agent,

   whereby the sum of the percentages of the components is 100.

5. Plaster according to one or more of the claims 1 to 4, characterized in that the pressure-sensitive hot melt adhesive is present in one or more layers, whereby the individual layers may be of different composition.

6. Plaster according to one or more of claims 1 to 5, characterized in that it comprises an active substance-impermeable, flexible backing layer, an active substance reservoir connected thereto which is formed of the pressure-sensitive hot melt adhesive, and, where other controlling mechanisms are not present, a membrane controlling the release of active substance, and optionally a pressure-sensitive adhesive device connected to the membrane for fixing the system to the skin, and a protective layer covering the skin-facing adhesive surface of the plaster and being detachable prior to application of the system.

7. Process for the production of the plaster according to claims 1 to 6, characterized by the following process steps:

   a) resin portions of the formulation are melted at approx. 60 to 200 °C,

b) polymers are slowly stirred into the melt until a clear melt is obtained,

c) subsequently or simultaneously, auxiliary substances and/or fillers are slowly added while stirring, until a homogeneous distribution is obtained (melt A),

d) in a separate stirring vessel dexpanthenol is melted at 50 °C and estradiol is slowly added in portions while stirring, until a clear solution is obtained (melt B),

e) melt B is slowly stirred into melt A, until a homogeneous mixture is obtained,

f) using a knife coating technique, the pressure-sensitive hot melt adhesive is thereafter spread at 60 to 100 °C onto a siliconised polyester protective layer (thickness 30 to 250 $\mu$m) such that a weight per area of 80 to 500 g/m$^2$, preferably 150 to 280 g/m$^2$, is obtained; after cooling down, a backing layer of polyester (thickness 6 to 100 $\mu$m) is laminated onto the pressure-sensitive hot melt adhesive,

g) subsequently, patches having an area of 10 cm$^2$ are punched out.

## Revendications

1. Emplâtre autocollant pour l'application transdermique d'hormones stéroïdiennes à activité systémique, comprenant une couche dorsale imperméable à la substance active, une couche auto-adhésive contenant la substance active et une couche de protection amovible recouvrant cette dernière avant l'application sur la peau, caractérisé en ce que la couche auto-adhésive contenant la substance active contient un agent auto-adhésif à fusion contenant, à titre de polymère, un copolymère d'éthylène-acétate de vinyle possédant une teneur en acétate de vinyle de 20 à 35%, dont la température de traitement s'élève entre 60 et 100°C, et du Dexpanthénol en une concentration de 15 à 25% en poids.

2. Emplâtre selon la revendication 1, caractérisé en ce que l'hormone stéroïdienne est l'oestradiol.

3. Emplâtre selon la revendication 1 ou 2, caractérisé en ce que l'agent auto-adhésif à fusion contient une résine tendre, une résine dure, des matières de remplissage et des agents de protection contre le vieillissement, à titre de substances auxiliaires.

4. Emplâtre selon une ou plusieurs des revendications 1 à 3, caractérisé par une composition de l'agent auto-adhésif à fusion possédant les constituants ci-après:

   à concurrence de 10 à 30% en poids, un copolymère d'éthylène-acétate de vinyle possédant une teneur en acétate de vinyle de 28%,

   à concurrence de 15 à 45% en poids, une résine tendre dont la viscosité en fusion est inférieure à 10 Pa.s à 60°C,

   à concurrence de 10 à 30% en poids, une résine d'hydrocarbure aliphatique dont la viscosité en fusion est supérieure à 10 Pa.s à 100°C,

   à concurrence de 15 à 25% en poids, du Dexpanthénol,

   à concurrence de 0,1 à 10% en poids, une hormone stéroïdienne,

   à concurrence de 0 à 5% en poids, une matière de remplissage,

   à concurrence de 0 à 5% en poids, un agent de protection contre le vieillissement,

   la somme des fractions en pour cent des constituants étant égale à 100.

5. Emplâtre selon une ou plusieurs des revendications 1 à 4, caractérisé en ce que l'agent auto-adhésif à fusion est présent dans une ou dans plusieurs couches, les couches individuelles pouvant être de compositions différentes.

6. Emplâtre selon une ou plusieurs des revendications 1 à 5, caractérisé en ce qu'il comprend une couche dorsale flexible imperméable à la substance active, un réservoir de la substance active lié à cette dernière et constitué par l'agent auto-adhésif à fusion, en l'absence d'autres mécanismes de commande, une membrane commandant la libération de la substance active et, le cas échéant, un mécanisme d'auto-adhésion relié à la membrane pour fixer le système sur la peau, et une couche de protection recouvrant la surface collante de l'emplâtre, orientée vers la peau et apte à être retirée avant l'application du système.

7. Procédé pour la fabrication de l'emplâtre selon les revendications 1 à 6, caractérisé par les étapes opératoires consistant à:

a) porter à fusion des fractions de résine de la formulation à une température d'environ 60 à 200°C,

b) tout en agitant, introduire lentement des polymères dans la masse fondue jusqu'à ce que l'on obtienne une masse fondue claire,

c) tout en agitant, introduire lentement par la suite ou simultanément des adjuvants et/ou des substances de remplissage jusqu'à ce que l'on obtienne une distribution uniforme (masse fondue A),

d) dans un récipient d'agitation séparé, porter à fusion du Dexpanthénol à une température de 50°C, introduire lentement dans la masse fondue de l'oestradiol par portions tout en agitant et, en fait, jusqu'à ce que l'on obtienne une solution claire (masse fondue B),

e) tout en agitant, introduire lentement la masse fondue B dans la masse fondue A jusqu'à ce que l'on obtienne un mélange intime uniforme,

f) appliquer ensuite l'agent auto-adhésif à fusion à une température de 60 à 100°C, en utilisant une racle, à raison d'un poids superficiel de 80 à 500 g/m$^2$, de préférence de 150 à 280 g/m$^2$ sur une couche de protection de polyester siliconisée (épaisseur 30 à 250 $\mu$m) et, après le refroidissement, contrecoller une couche dorsale de polyester (épaisseur 6 à 100 $\mu$m) sur l'agent auto-adhésif à fusion,

g) ensuite, découper des emplâtres à la matrice pour obtenir une surface de 10 cm$^2$.